# EUROPEAN PATENT SPECIFICATION

(11) **EP 2 213 222 B1**
(45) Date of publication and mention of the grant of the patent: **28.11.2018**
(21) Application number: 10151752.2
(22) Date of filing: 27.01.2010
(51) Int. Cl.: A61B 1/00, A61B 1/04, A61B 1/06, A61B 5/00, G01N 21/64

(54) **Fluorescence endoscope system**
Fluoreszenzendoskopiesystem
Système endoscope à fluorescence

(30) Priority: 30.01.2009 JP 2009019403
(43) Date of publication of application: 04.08.2010
(73) Proprietor: FUJIFILM Corporation, Minato-ku Tokyo (JP)
(72) Inventor: KOMUKAI, Makito, Saitama-city Saitama (JP)
(74) Representative: Klunker IP Patentanwälte PartG mbB

(56) References cited:
- US-A- 4 786 813
- US-A- 6 070 096
- US-A1- 2008 039 695

## Description

### BACKGROUND OF THE INVENTION

### 1. Field of the Invention

The present invention relates to a fluorescence endoscope system for observing an internal body part labeled by a fluorescent dye.

### 2. Description Related to the Prior Art

Conventionally, fluorescence endoscopy using a fluorescent dye or a fluorescent substance is known. In the fluorescence endoscopy, a fluorescent labeling agent that contains the fluorescent dye and is selectively bonded to a lesion is administered on an internal body part. After the administration of the fluorescent labeling agent, the internal body part is irradiated with an excitation light, and is observed in a fluorescence image to find out the lesion including a cancer, a tumor, and ischemia that is difficult to find out in a normal visible-light endoscope image. However, living body tissue originally contains an autofluorescent material, and shows autofluorescence by the irradiation with the excitation light. The autofluorescence becomes noise in the fluorescence endoscopy using the fluorescent dye. Consequently, it has been required to reduce the effect of the autofluorescence.

United States Patent Application Publication No. 2008/0039695 (corresponding to Japanese Patent Laid-Open Publication No. 2008-043494) proposes a fluorescence endoscope system that reduces the effect of autofluorescence. This system has an excitation-light radiating part for radiating an excitation light having a wavelength within an absorption spectrum of a fluorescent dye, and a correction-information acquiring part. The excitation-light radiating part applies the excitation light to an internal body part before administration of a fluorescent labeling agent, in order to obtain an autofluorescence image. The correction-information acquiring part obtains correction information from the autofluorescence image.

Then, the correction information is subtracted from an observation fluorescence image that is captured after the administration of the fluorescent labeling agent by irradiation with the excitation light. The observation fluorescence image includes both of fluorescence from the fluorescent dye and autofluorescence from an autofluorescent material. Accordingly, subtracting the light intensity of the autofluorescence image from that of the observation fluorescence image allows elimination of noise due to the autofluorescence.

However, in the case of observing a plurality of adjacent areas on a continual basis, a fluorescent labeling agent that has been administered on a former observation area likely spatters on the next observation area unintentionally. In this case, a fluorescent dye (fluorescent probe) that is accidentally spattered on and bonded to a lesion shows fluorescence, and is wrongly measured as autofluorescence. This causes to get inaccurate correction information.

US 4,786,813 A discloses a fluorescence endoscope system in which an actually picked-up image is compared with a standard image previously registered in a computer memory in order to accept or to reject the object of the image dependent on the extent of deviation between the two compared images.

US 6,070,096 A discloses a fluorescence detecting apparatus. A deceased part of a living body is detected by forming a quotient of a first and a second intrinsic fluorescence component.

### SUMMARY OF THE INVENTION

An object of the present invention is to provide a fluorescence endoscope system that can appropriately eliminate noise of autofluorescence.

A fluorescence endoscope system according to the present invention includes the features of claim 1.

The endoscope insert section has an image taking unit at its distal end. The excitation light from the light source unit is radiated from the distal end of the endoscope insert section. Before capture of an autofluorescence image, the light source unit applies the excitation light to an observation area to carry out a fluorescence quenching mode. The image capture control section captures a fluorescence image of the observation area at twice at a predetermined time interval during the fluorescence quenching mode. The captured two fluorescence images are written to the memory. The image processing circuit reads out the two fluorescence images from the memory, compares light intensity between the two fluorescence images, and output a difference signal. The judgment circuit judges whether or not fluorescence quenching of the fluorescent dye that already existed in the observation area has been completed based on the difference signal. After the completion of the fluorescence quenching, the autofluorescence image is captured.

It is preferable that the observation area be continuously irradiated with the excitation light for a predetermined time before capture of the two fluorescence images.

According to the present invention, before the observation area inside the body cavity is specifically stained by the fluorescent dye, the observation area is irradiated with the excitation light, and the fluorescence image is taken twice at the predetermined time interval. The light intensity of the two fluorescence images is compared to judge whether or not fluorescence from the fluorescent dye that already existed in the observation area has been quenched. Since the autofluorescence image is captured after the completion of the fluorescent quenching, it is possible to obtain accurate correction information without the effect of the fluorescent dye.

### BRIEF DESCRIPTION OF THE DRAWINGS

For more complete understanding of the present invention, and the advantage thereof, reference is now made to the following descriptions taken in conjunction with the accompanying drawings, in which:
Fig. 1 is a circuit block diagram of a fluorescence endoscope system;
Fig. 2 is an explanatory view showing the general outline of an image taking unit of the fluorescence endoscope system;
Fig. 3 shows waveform diagrams of spectral transmittance of a variable filter spectrometer in first and second states, spectral transmittance of an excitation light cut filter, spectral intensity of an excitation light, spectral intensity of a normal light, spectral intensity of fluorescence from a fluorescent dye, and spectral intensity of fluorescence of an observation area;

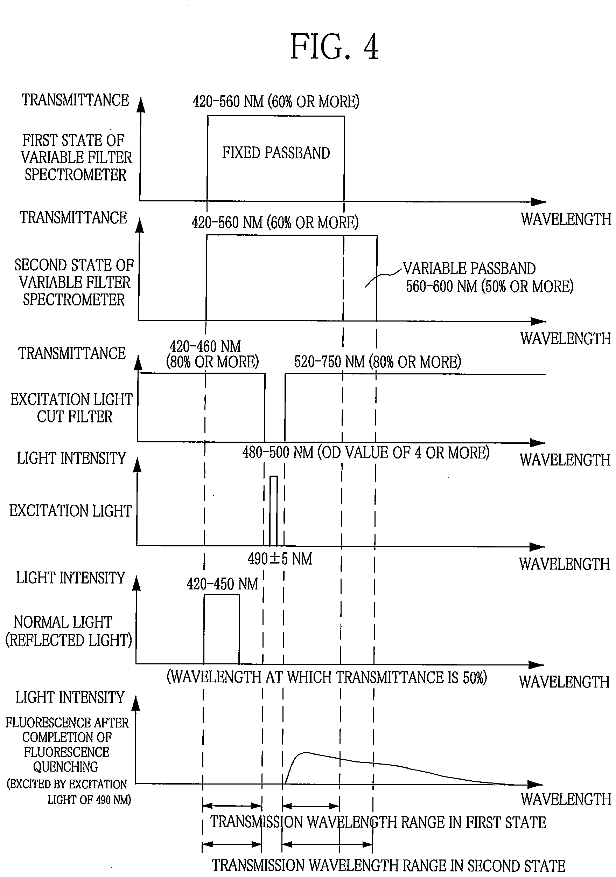

Fig. 4 shows waveform diagrams similar to Fig. 3 after completion of fluorescence quenching;
Fig. 5 is a timing chart that explains the operation of the fluorescence endoscope system;
Fig. 6 is a flowchart of the operation of the fluorescence endoscope system;
Fig. 7 is a graph that shows a state of attenuation of fluorescence under irradiation with an excitation light; and
Fig. 8 is a flowchart of the operation of a fluorescence endoscope system.

### DESCRIPTION OF THE PREFERRED EMBODIMENTS

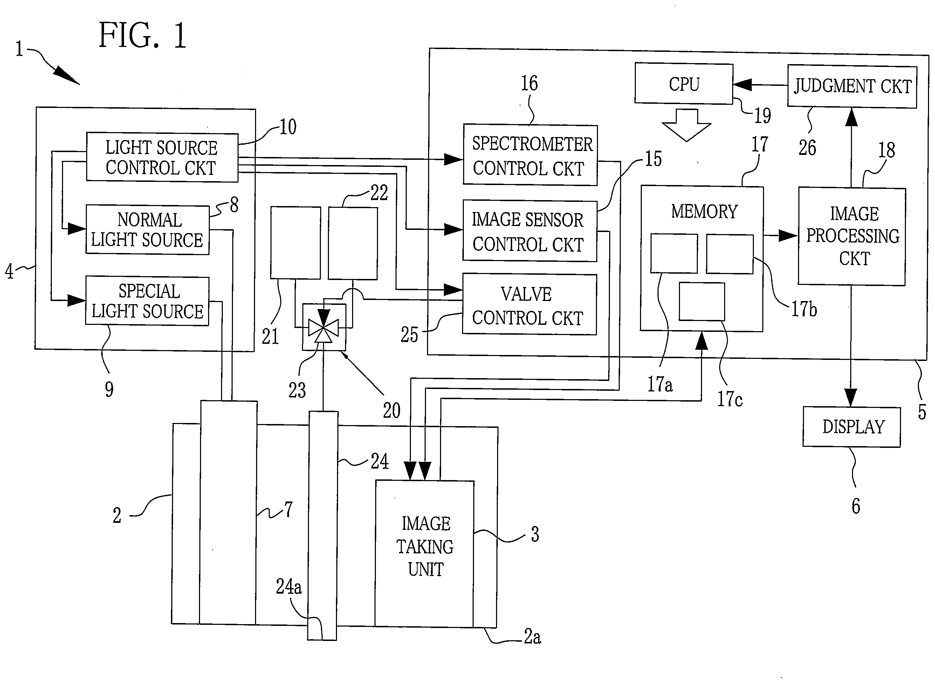

A fluorescence endoscope system 1, as shown in Fig. 1, is provided with an endoscope insert section 2 introduced into a human body cavity, an image taking unit 3 disposed in the endoscope insert section 2, a light source unit 4 for having plural types of light sources, a liquid delivery unit 20, a control unit 5, and a display 6. The liquid delivery unit 20 selectively discharges liquids contained in first and second tanks 21 and 22 from a distal end 24a of a liquid feed tube 24 of the endoscope insert section 2. The control unit 5 controls the image taking unit 3, the light source unit 4, and the liquid delivery unit 20. On the display 6, an image captured by the image taking unit 3 is displayed.

The long slender endoscope insert section 2 has an extremely small diameter of, for example, approximately 5mm, and is introduced into the human body cavity from patient's mouth, nose, or the like. In the endoscope insert section 2, there are provided the image taking unit 3, a light guide 7 for transmitting light from the light source unit 4 to the distal end 2a of the endoscope insert section 2, and a liquid feed tube 24 for conveying the liquids from the liquid delivery unit 20. The light source unit 4 has a normal light source 8 for emitting a normal light, a special light source 9 for emitting an excitation light, and a light source control circuit 10 for controlling the normal light source 8 and the special light source 9. The normal light from the normal light source 8 is applied to an observation area, an imaging area, or a target site inside the body through the light guide 7, and a reflected light is captured by the image taking unit 3. In a like manner, the excitation light from the special light source 9 is applied to the observation area through the light guide 7, and excites a fluorescent substance such as a fluorescent dye existing therein to generate fluorescence.

The normal light source 8 is a combination of, for example, a xenon lamp and a band-pass filter, both of which are not illustrated. The band-pass filter is set up to transmit a light having a wavelength of 420 to 450 nm at a transmittance of 50%. Thus, the normal light is a visible light in a wavelength band of 420 to 450 nm.

The special light source 9 is, for example, a semiconductor laser for emitting a laser light having a peak wavelength of 490±5 nm as the excitation light. This excitation light excites an esterase-sensitive fluorescent dye having a fluorescein skeleton. Fluorescence endoscopy with the fluorescence endoscope system 1 uses a fluorescent labeling agent that contains this esterase-sensitive fluorescent dye and is selectively bonded to tissue of a tumor. As the special light source 9, an argon laser that emits a laser light of 488±5 nm is also available instead of the semiconductor laser.

As such a fluorescent dye (fluorescent probe) or a fluorophore that is exceptionally bonded to the lesion, there are, for example, fluorescein esters including fluorescein diacetate (FDA) . These fluorescent dyes have the property of fluorescence quenching by light excitation. Instead of above, another fluorescent dye is available as long as it has the property of fluorescence quenching. As used herein, the term "fluorescence quenching" means reduction in fluorescence due to photochemical destruction of the fluorescent substance by irradiation with the excitation light.

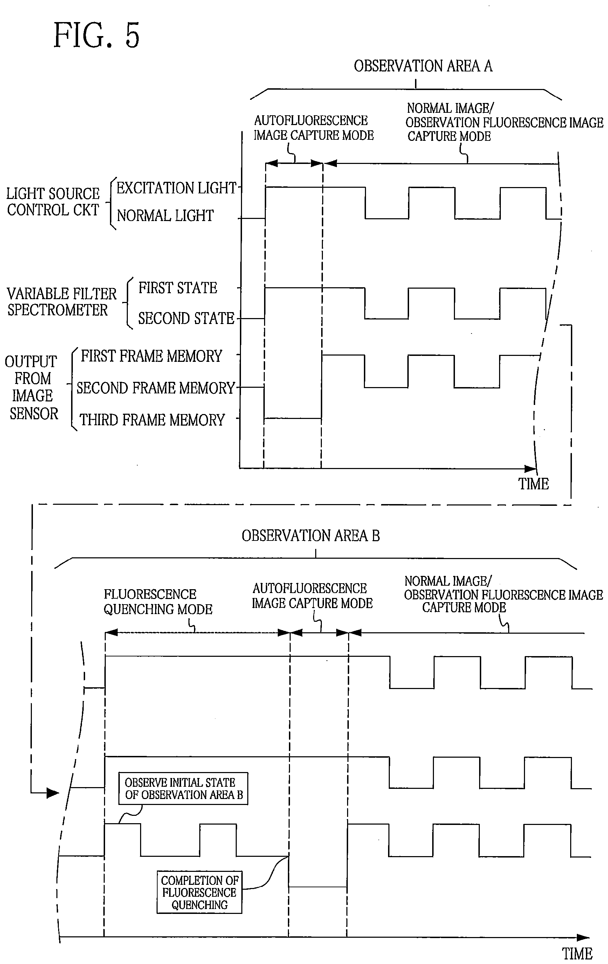

The light source control circuit 10, as shown in Fig. 5, alternately turns on and off the normal light source 8 and the special light source 9 in predetermined cycles.

The control unit 5 includes an image sensor control circuit 15, a spectrometer control circuit 16, a valve control circuit 25, a memory 17, and an image processing circuit 18. The image sensor control circuit 15 controls actuation of an image sensor 14 disposed in the image taking unit 3. The spectrometer control circuit 16 controls actuation of a variable filter spectrometer 13 disposed in the image taking unit 3. The memory 17 stores an image captured by the image sensor 14. The image processing circuit 18 applies image processing to the image stored in the memory 17, and outputs the image to the display 6. The image sensor control circuit 15, the spectrometer control circuit 16, the valve control circuit 25, the memory 17, and the image processing circuit 18 are overall controlled by a CPU 19.

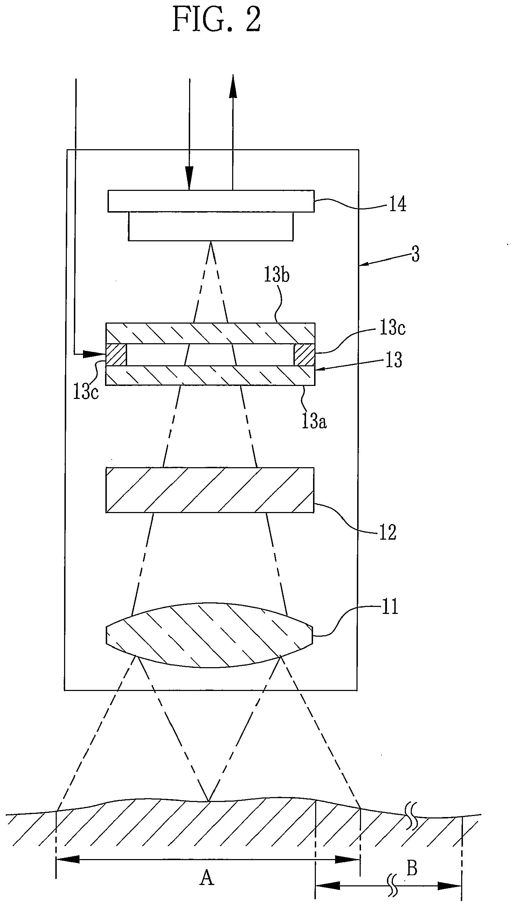

As shown in Fig. 2, the image taking unit 3 has an imaging lens system 11, an excitation light cut filter 12, the variable filter spectrometer 13, and the image sensor 14. The imaging lens system 11 gathers light incident from an observation area A inside the body. The excitation light cut filter 12 intercepts the excitation light of the light incident upon the imaging lens system 11. The variable filter spectrometer 13 has a passband that is variable by the spectrometer control circuit 16. The image sensor 14 captures light that has passed through the imaging lens system 11, the excitation light cut filter 12, and the variable filter spectrometer 13, and converts the light into an electric signal.

The variable filter spectrometer 13, being an etalon optical filter, has two plate-shaped optical elements 13a and 13b and an actuator 13c. The optical elements 13a and 13b are disposed in parallel with each other with leaving a predetermined clearance. The actuator 13c varies the amount of the clearance between the optical elements 13a and 13b. A top face of the optical element 13a and a bottom face of the optical element 13b are provided with a reflective film. The actuator 13c is, for example, a piezoelectric element. When the actuator 13c widens or narrows the clearance between the optical elements 13a and 13b, the passband of the variable filter spectrometer 13 is varied.

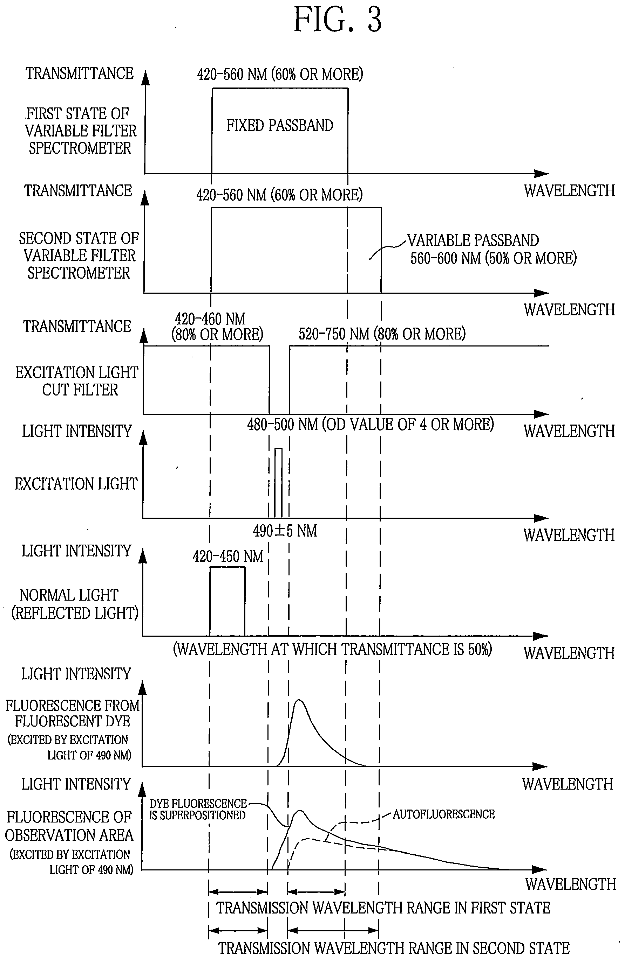

The variable filter spectrometer 13, as shown in Fig. 3, has a single fixed passband and a single variable passband. The fixed passband is set at 420 to 560 nm with a transmittance of 60% or more.

The variable passband is in a red wavelength band, for example, 560 to 600 nm. The variable filter spectrometer 13 is switchable between a first state and a second state in response to a control signal from the control unit 5.

In the first state, the transmittance of light in the variable passband is set sufficiently smaller than that in the second state. In other words, the light in the variable passband is hardly transmitted in the first state, though light in the fixed passband is transmitted. The spectrum of fluorescence from the fluorescent dye is mainly in a range of 520 to 560 nm, as shown in Fig. 3, so that the fluorescence from the fluorescent dye is sufficiently transmitted in the first state.

In the second state, the transmittance of the variable passband is set at 50% or more. In other words, light in both of the fixed passband and the variable passband is transmitted in the second state, and hence all of blue light, green light, and red light, which are required to observe white, are transmitted. The normal light has a wavelength of, for example, 420 to 450 nm to adequately depict information of a blood vessel. Otherwise, a red light (580 to 590 nm) that has a low absorptance into body tissue and more clearly depicts the surface shape of an organ than the blue light may be used as the normal light.

The excitation light cut filter intercepts light with a wavelength of 480 to 500 nm. The transmittance of the excitation light cut filter 12 is 80% or more at a wavelength of 420 to 470 nm, 1 × 10⁻⁴ or less (optical density (OD) value of 4 or more) at a wavelength of 480 to 500 nm, and 80% or more at a wavelength of 520 to 750 nm.

In an endoscopic examination, a target site is divided into a plurality of areas, and images of individual areas are successively observed. Fig. 2 takes a case where adjacent areas A and B are successively observed as an example. In the endoscopic examination, an autofluorescence image of the area A is first taken, and then the fluorescent labeling agent is administered thereon. After the administration of the fluorescent labeling agent, an observation fluorescence image is taken. As shown in Fig. 3, the light intensity of the observation fluorescence image is the superposition of the light intensity of dye fluorescence and the light intensity of the autofluorescence. Accordingly, subtracting the light intensity of the autofluorescence image from that of the observation fluorescence image can obtain a fluorescence image having light intensity of the fluorescent dye.

In observation of the next area B, however, the fluorescent labeling agent that has been administered on the area A is often spattered on the area B by overflow. If an autofluorescence image is taken in this state, autofluorescence cannot be accurately measured because the autofluorescence image contains fluorescence from the fluorescent dye. In this case, the fluorescence from the fluorescent dye that is exceptionally bonded to the lesion is wrongly recognized as the autofluorescence, causing overlook of the lesion. For this reason, according to the present invention, the autofluorescence image is captured after confirming completion of fluorescence quenching of the fluorescent dye. To realize it, there are two ways, that is, to confirm whether or not an image has been taken after the completion of the fluorescence quenching, and to take an image after completion of the fluorescence quenching has been confirmed. In this example, an autofluorescence image is obtained by confirming whether or not the autofluorescence image has been taken after completion of the fluorescence quenching.

As illustrated in Fig. 1, the image sensor control circuit 15 and the spectrometer control circuit 16 are connected to the light source control circuit 10. The CPU 19 switches the variable filter spectrometer 13 between the first state and the second state, in synchronization with the switching between the normal light source 8 and the special light source 9. The CPU 19 controls the image sensor control circuit 15 and the spectrometer control circuit 16 so that the single image sensor 14 captures images of an observation area that is irradiated with plural types of lights in predetermined order, and the captured images are stored on first to third frame memories 17a to 17c of the memory 17.

The fluorescence endoscope system 1, as shown in Fig. 5, carries out an autofluorescence image capture mode, a normal image/observation fluorescence image capture mode, and a fluorescence quenching mode. In the autofluorescence image capture mode, the excitation light is emitted from the special light source 9, and the variable filter spectrometer 13 is set in the first state. The image sensor 14 captures an autofluorescence image of the observation area, and the autofluorescence image is stored on the third frame memory 17c. This autofluorescence image is based on a fluorescent material contained in living body tissue, and is produced under irradiation with the excitation light in a state without existence of the fluorescent dye or after completion of the fluorescence quenching of the fluorescent dye.

The normal image/observation fluorescence image capture mode is carried out, after the fluorescent labeling agent is administered on the observation area. In this mode, the observation area is alternately irradiated with the normal light and the excitation light in predetermined cycles, to capture normal images (reflected light images) and observation fluorescence images. When the excitation light is emitted from the special light source 9, the variable filter spectrometer 13 is set in the first state. The image sensor 14 captures the observation fluorescence image of the observation area, and the observation fluorescence image is stored on the first frame memory 17a. The observation fluorescence image contains the dye fluorescence by the administered fluorescent labeling agent and the autofluorescence. When the normal light is emitted from the normal light source 8, on the other hand, the variable filter spectrometer 13 is set in the second state. The image sensor 14 captures the normal image, and the normal image is stored on the second frame memory 17b.

The image processing circuit 18 reads the observation fluorescence image from the first frame memory 17a, and reads the autofluorescence image from the third frame memory 17c. The image processing circuit 18 subtracts the light intensity of the autofluorescence image from that of the observation fluorescence image, and outputs a corrected fluorescence image from which noise of the autofluorescence is removed.

The fluorescence quenching mode is a mode to quench the fluorescence emission from the fluorescent dye existing in the observation area. In this mode, as shown in a lower part of Fig. 5, the excitation light is emitted from the special light source 9, and the variable filter spectrometer 13 is set in the first state. The image sensor 14 captures two images at a predetermined time interval. The first image outputted from the image sensor 14 is stored on the first frame memory 17a as a former fluorescence image. The second image is stored on the second frame memory 17b as a latter fluorescence image.

The image processing circuit 18 reads the former fluorescence image from the first frame memory 17a and reads the latter fluorescence image from the second frame memory 17b, and calculates subtraction in the light intensity between the former fluorescence image and the latter fluorescence image. The data of the subtraction in the light intensity is sent to a judgment circuit 26. The judgment circuit 26 judges that the fluorescence quenching is in progress, if the subtraction is not almost zero. The judgment circuit 26 judges that the fluorescence quenching has been completed, if the subtraction is almost zero. The CPU 19 decides, based on a judgment result of the judgment circuit 26, whether or not to carry on the fluorescence quenching mode, in other words, whether or not to shift operation to the autofluorescence image capture mode.

If there is a difference in light intensity, the fluorescence quenching mode is continued. Taking a case where the fluorescence quenching mode is repeated at "n" times ("n" is an arbitrary natural number) for example, as with above, a (2n-1) th former fluorescence image is stored on the first frame memory 17a, a (2n) th latter fluorescence image is stored on the second frame memory 17b. The image processing circuit 18 calculates subtraction in light intensity between the former fluorescence image and the latter fluorescence image. The judgment circuit 26 judges a state of fluorescence quenching from the subtraction.

If the light intensity of the two fluorescence images is judged to be equal, the fluorescence endoscope system 1 is shifted from the fluorescence quenching mode to the autofluorescence image capture mode. The fluorescence images inevitably have noise. Thus, the difference in light intensity does not necessarily have to be zero. The fluorescence quenching is judged to be completed, if the difference in light intensity is a predetermined threshold value or less.

The liquid delivery unit 20 is provided with the first tank 21, the second tank 22, a valve 23, the liquid feed tube 24 disposed in the endoscope insert section 2, and the valve control circuit 25 disposed in the control unit 5. The first tank 21 stores water for rinsing the observation area or the target site. The second tank 22 stores the fluorescent labeling agent. The valve 23 selectively feeds or stops the water and the fluorescent labeling agent from the tanks 21 and 22 to the liquid feed tube 24. The valve 23 is constituted of, for example, a three way valve. The water or the fluorescent labeling agent fed through the valve 23 and the liquid feed tube 24 is administered from the distal end 2a of the endoscope insert section 2 on the observation area. As the liquid feed tube 24, a forceps channel provided in the endoscope insert section 2 may be used.

The valve control circuit 25 is connected to the light source control circuit 10. The valve control circuit 25 carries out at least the operation of administering the fluorescent labeling agent from the second tank 22 in synchronization with the emission of the excitation light from the special light source 9.

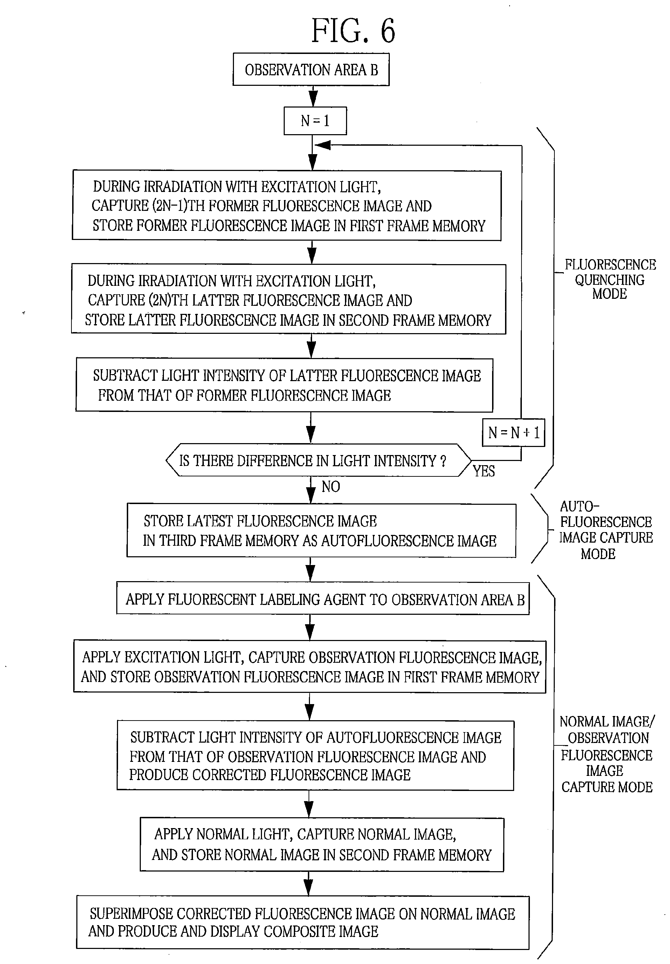

Next, the operation of the fluorescence endoscope system 1 will be described with referring to Fig. 6. To carry out a fluorescence endoscope examination, the fluorescence endoscope system 1 is turned on, and the endoscope insert section 2 is introduced into the human body cavity. The distal end 2a of the endoscope insert section 2 is faced toward the first observation area A (for example, a portion suspected to be a lesion).

In observation of the observation area A, the autofluorescence image capture mode, the administration of the fluorescent labeling agent, and the normal image/observation fluorescence image capture mode are carried out in this order. In the first observation area A, since the autofluorescence image is captured before the administration of the fluorescent labeling agent, there is no need to carry out the fluorescence quenching mode. After the observation of the observation area A, observation of the observation area B that is positioned next to the observation area A is carried out. On the observation area B, the fluorescent labeling agent is likely spattered unintentionally.

Thus, in the observation of the observation area B, the fluorescence quenching mode is first carried out. In this mode, the excitation light is transmitted from the special light source 9 through the light guide 7 to the distal end 2a of the endoscope insert section 2, and is applied to the observation area B. Fluorescence generated on the observation area B is gathered by the imaging lens system 11 of the image taking unit 3, and is incident on the variable filter spectrometer 13 through the excitation light cut filter 12.

The variable filter spectrometer 13 is set in the first state in synchronization with the operation of the special light source 9 by control of the spectrometer control circuit 16. Consequently, the fluorescence generated on the observation area B is transmitted at a high transmittance of 60% or more. At this time, a part of the excitation light is reflected from the adjacent observation area A, and is incident on the image taking unit 3 together with the fluorescence. The excitation light, however, is intercepted by the excitation light cut filter 12 of the image taking unit 3, and hence is not incident on the image sensor 14.

The fluorescence that has passed through the variable filter spectrometer 13 is captured by the image sensor 14, and is written to the first frame memory 17a as the former fluorescence image. After that, while the special light source 9 keeps emitting the excitation light, the image sensor 14 captures the next image at the predetermined time interval. This image is written to the second frame memory 17b as the latter fluorescence image. The image processing circuit 18 reads the former fluorescence image from the first frame memory 17a, and reads the latter fluorescence image from the second frame memory 17b. The image processing circuit 18 calculates a difference in light intensity between the former and latter fluorescence images, and sends a light intensity difference signal to the judgment circuit 26.

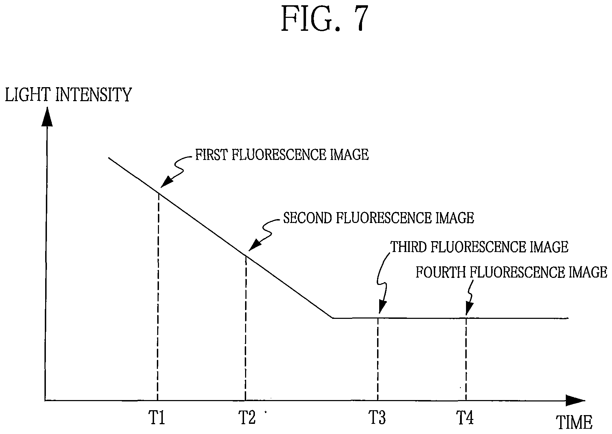

The judgment circuit 26 judges whether or not there is a difference in light intensity between the two fluorescence images. Taking the case of Fig. 7 as an example, a difference in light intensity is calculated between a first fluorescence image captured at time t1 and a second fluorescence image captured at time t2. In this example, there is a difference in light intensity between the time t1 and the time t2. As a result, it is judged that the fluorescent dye existing in the observation area has not been quenched yet, and hence the fluorescence quenching mode is carried on. After the excitation light has been applied for predetermined time, a third fluorescence image is captured at time t3. Then, a fourth fluorescence image is captured at time t4. Since there is no difference in light intensity between the time t3 and the time t4, it is judged that the fluorescence quenching has been completed. After this judgment, the operation is shifted from the fluorescence quenching mode to the autofluorescence image capture mode.

In the autofluorescence image capture mode, the image that is captured at the end of the fluorescence quenching mode, that is, the fourth fluorescence image captured at the time t4 of Fig. 7 is written to the third frame memory 17c as an autofluorescence image.

After that, the fluorescent labeling agent is administered on the observation area B. To administer the fluorescent labeling agent, the CPU 19 controls the valve control circuit 25 so as to switch the valve 23 to the second tank 22. Accordingly, the fluorescent labeling agent stored in the second tank 22 is discharged from a distal end 24a of the liquid feed tube 24 toward the observation area B.

While the fluorescent labeling agent is administered, the special light source 9 is actuated to apply the excitation light to the observation area B. Thus, if the fluorescent labeling agent is colorless, the fluorescent labeling agent can be certainly administered with confirmation of an administered condition. After a lapse of reaction time of the fluorescent labeling agent from the administration thereof, the fluorescence endoscope system 1 is shifted to the normal image/observation fluorescence image capture mode.

The observation fluorescence image captured after the administration of the fluorescent labeling agent is written to the first frame memory 17a. Then, the light intensity of the autofluorescence image, which has been stored on the third frame memory 17c, is subtracted from that of the observation fluorescence image, for the purpose of obtaining the corrected fluorescence image. The corrected fluorescence image is an image in which noise due to the autofluorescence is removed from the observation fluorescence image of the observation area B, i.e. an image based on the dye fluorescence.

Subsequently, the normal light is applied to the observation area B. At this time, the variable filter spectrometer 13 is switched into the second state. The normal light is reflected from the surface of the observation area B. The reflected normal light is gathered by the imaging lens system 11, and is incident on the variable filter spectrometer 13 through the excitation light cut filter 12. Since the wavelength band of the reflected normal light is within the passband of the variable filter spectrometer 13, all of the reflected normal light passes through the variable filter spectrometer 13.

The reflected normal light that has passed through the variable filter spectrometer 13 is incident on the image sensor 14, and is captured as the normal image (visible light image). This normal image is written to the second frame memory 17b. The image processing circuit 18 produces a composite image in which the corrected fluorescence image is superimposed on the normal image, and the composite image is outputted to the displayed 6.

As described above, according to this example, since the autofluorescence image is captured after the confirmation of the fluorescence quenching of the fluorescent dye existing in the observation area, accurate correction information is obtained. Therefore, it is possible to certainly remove noise due to the autofluorescence, and observe the fluorescence from the fluorescent dye of the lesion with high precision.

In the foregoing example, fluorescein is used as the fluorescent dye. Fluorescence of the fluorescein is quenched for a little less than one minute, when being excited by an excitation light of about 20 mW. Thus, the fluorescence quenching mode is finished within tens of seconds. The physical properties of collagen, which is a main generation source of the autofluorescence, are not changed by irradiation with the light. Thus, the autofluorescence is not changed even if the excitation light has been emitted for tens of seconds.

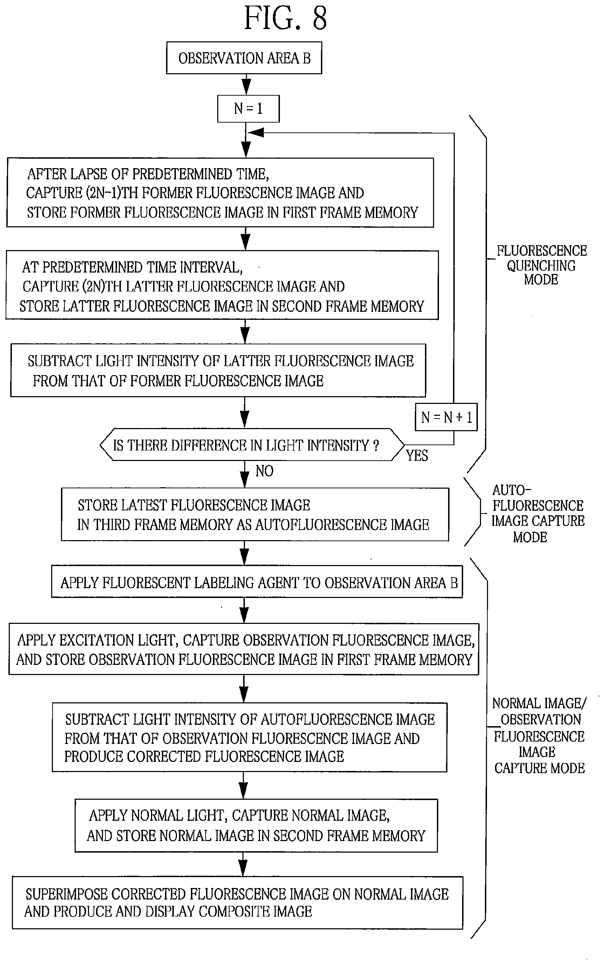

In the foregoing fluorescence quenching mode, the quenching of fluorescence from the fluorescent dye is accelerated by irradiation with the excitation light, and the fluorescence from the fluorescent dye disappears within, for example, tens of seconds. Accordingly, the relation between the amount of administered fluorescent labeling agent and the radiation amount of excitation light can be investigated in advance, and the state of fluorescence quenching is judged by irradiation time with the excitation light. In this case, as shown in Fig. 8, the former fluorescence image and the latter fluorescence image may be captured after a lapse of predetermined time.

The number of photons that are emitted from a general fluorescent dye is 10⁵ to 10⁶ at most. This fluorescent dye shows fluorescence having a wavelength of 500 nm and an output of 200 mW, and the fluorescence is quenched within 5 to 50 seconds. Consequently, if the two fluorescence images are captured to compare their light intensity before and after a lapse of predetermined time or after the lapse of predetermined time during irradiation with the excitation light, operation is smoothly shifted to the next mode without repeating measurement.

In the fluorescence endoscope system 1 according to this example, as shown in Fig. 5, normal light observation is carried out by the operation of the light source control circuit 10 and the valve control circuit 25, in advance of the first step in the autofluorescence image capture mode. In the normal light observation, the normal light (visible light) is applied from the normal light source 8 to the observation area A.

Upon switching from the normal observation to the autofluorescence image capture mode, the valve control circuit 25 switches the valve 23 to the first tank 21, while the normal light source 8 emits the normal light in advance of emission of the excitation light. Accordingly, the water stored in the first tank 21 is discharged from the distal end 24a of the liquid feed tube 24 toward the observation area A to clean the surface of the observation area A. After that, in the autofluorescence image capture mode, the autofluorescence image is captured under irradiation with the excitation light, and then the fluorescent labeling agent containing the esterase-sensitive fluorescent dye is administered on the observation area A. Therefore, a target site suspected to be a lesion is labeled by the esterase-sensitive fluorescent dye (fluorescent probe), and is confirmed whether or not to be a lesion.

In the foregoing example, the imaging lens system 11, the excitation light cut filter 12, and the variable filter spectrometer 13 are disposed in this order from the side of the distal end 2a of the endoscope insert section 2 in the image taking unit 3, but disposition order thereof is appropriately changeable.

It is medically known that esterase of a higher amount is present on tumor tissue than on normal tissue. Fluorescence is emitted more rapidly at the tumor tissue upon reaction of the fluorescein. Thus, fluorescence of a higher intensity can be detected from the tumor tissue.

A fluorescent compound of the present example is fluorochrome or fluorophore having a molecular structure or skeleton reacting specifically with affected tissue. The fluorescent compound may be substances other than fluorescein or its derivatives . It is possible in the invention to use a material produced by labeling of an antibody or the like with a fluorescent compound, and to detect affected tissue immunologically.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. A fluorescence endoscope system (1) for imaging an observation area specifically stained by a fluorescent dye inside a body cavity, comprising:
an endoscope insert section (2) to be introduced into the body cavity, and having an image taking unit (3) at a distal end (2a) thereof;
a light source unit (4) including a special light source (9) that is configured to emit an excitation light of wavelength included in an absorption spectrum of the fluorescent dye, the excitation light being applied from the distal end of the endoscope insert section to the observation area before the observation area is stained by the fluorescent dye;
an image capture control section adapted to capture a fluorescence image of the observation area at twice at a predetermined time interval by the image taking unit while the observation area is irradiated with the excitation light;
a memory (17) adapted to store the two fluorescence images;
an image processing circuit (18) adapted to compare light intensity of the two fluorescence images read out of the memory and to output a difference signal; **characterized by**
a judgment circuit (26) for judging whether or not fluorescence from the fluorescent dye that has already existed in the observation area is quenched based on the difference signal.

2. The fluorescence endoscope system (1) according to claim 1, wherein said light source unit is configured to irradiate the observation area continuously with the excitation light for a predetermined time before capture of the two fluorescence images.

## Patentansprüche

1. Fluoreszenz-Endoskopsystem (1) zum Abbilden eines Betrachtungsbereichs, der speziell durch einen Fluoreszenzfarbstoff im Inneren eines Körperhohlraums angefärbt ist, umfassend:
einen Endoskopeinführabschnitt (2) zum Einführen in den Körperhohlraum, ausgestattet mit einer Bildaufnahmeeinheit (3) an seinem distalen Ende (2a);
eine Lichtquelleneinheit (4) mit einer Speziallichtquelle (9), die konfiguriert ist zum Emittieren eines Anregungslichts einer Wellenlänge, die in einem Absorptionsspektrum des fluoreszierenden Farbstoffs enthalten ist, wobei das Anregungslicht von dem distalen Ende des Endoskopeinführabschnitts auf den Betrachtungsbereich aufgebracht wird, bevor der Betrachtungsbereich durch den fluoreszierenden Farbstoff angefärbt wird;
einen Bildaufnahme-Steuerabschnitt, ausgebildet zum Aufnehmen eines Fluoreszenzbilds des Betrachtungsbereichs zweimal während eines vorbestimmten Zeitintervalls durch die Bildaufnahmeeinheit, während der Betrachtungsbereich mit dem Anregungslicht bestrahlt wird;
einen Speicher (17), ausgebildet zum Speichern der beiden Fluoreszenzbilder;
eine Bildverarbeitungsschaltung (18), ausgebildet zum Vergleichen der Lichtintensität der beiden aus dem Speicher ausgelesenen Fluoreszenzbilder und zum Ausgeben eines Differenzsignals; **gekennzeichnet durch**:
eine Beurteilungsschaltung (26) zum Beurteilen, ob Fluoreszenz aus dem fluoreszierenden Farbstoff, der sich bereits in dem Betrachtungsbereich befunden hat, gelöscht wird oder nicht, basierend auf dem Differenzsignal.

2. System (1) nach Anspruch 1, bei dem die Lichtquelleneinheit konfiguriert ist zum kontinuierlichen Bestrahlen des Betrachtungsbereichs mit dem Anregungslicht für eine vorbestimmte Zeitspanne vor der Aufnahme der beiden Fluoreszenzbilder.

## Revendications

1. Système endoscopique à fluorescence (1) destiné à imager une zone d'observation colorée spécifiquement par un colorant fluorescent dans une cavité corporelle, comprenant :
une section d'introduction d'endoscope (2) à introduire dans la cavité corporelle, et présentant une unité de prise d'images (3) sur une extrémité distale (2a) de celle-ci ;
une unité de source lumineuse (4) incluant une source lumineuse spéciale (9), laquelle est configurée pour émettre une lumière d'excitation de longueur d'onde incluse dans un spectre d'absorption du colorant fluorescent, la lumière d'excitation étant appliquée à partir de l'extrémité distale de la section d'introduction d'endoscope à la zone d'observation avant que la zone d'observation ne soit colorée par le colorant fluorescent ;
une section de commande de capture d'image, apte à capturer une image de fluorescence de la zone d'observation par deux fois à l'intervalle de temps prédéterminé par l'unité de prise d'image tandis que la zone d'observation est irradiée avec la lumière d'excitation ;
une mémoire (17) apte à stocker les deux images de fluorescence ;
un circuit de traitement d'images (18) apte à comparer l'intensité lumineuse des deux images de fluorescence lues de la mémoire et à produire un signal de différence ;
**caractérisé par**
un circuit de jugement (26) pour juger si oui ou non une fluorescence émanant du colorant fluorescent ayant déjà existé dans la zone d'observation est désactivée sur la base du signal de différence.

2. Système endoscopique à fluorescence (1) selon la revendication 1, dans lequel ladite unité de source lumineuse est configurée pour irradier la zone d'observation en continu avec la lumière d'excitation pour un temps prédéterminé avant la capture des deux images de fluorescence.
